# EUROPEAN PATENT APPLICATION

(11) **EP 4 840 957 A1**
(43) Date of publication of application: **18.11.2026**
(21) Application number: 25305690.7
(22) Date of filing: 14.05.2025
(51) Int. Cl.: C12N 15/113

(54) **OLIGONUCLEOTIDES TARGETING TRISTETRAPROLIN MRNA AND THEIR USE IN THE TREATMENT/PREVENTION OF INFLAMMATORY DISEASES**

(71) Applicant: Université de Montpellier, 34090 Montpellier (FR); Centre National de la Recherche Scientifique, 75794 Paris Cedex 16 (FR); Institut National de la Santé et de la Recherche Médicale, 75013 Paris (FR)
(72) Inventor: TAULAN-CADARS, Magali, 34270 CAZEVIEILLE (FR); BLEUSE, Solenne, 16446 KISTA (SE)
(74) Representative: Ipsilon

(57) **Abstract**

The invention concerns an oligonucleotide (ASO) whose nucleotide sequence comprises, or consists of, a sequence having at least 90% identity, preferably 91%, 92%, 93%, 94%, 95%, 96%, 97%, 98%, 99% or 100% identity with a sequence selected from SEQ ID NO: 1 and SEQ ID NO: 2. It also relates to the use of this ASO for the prevention and/or treatment of inflammatory diseases, particularly inflammatory lung diseases such as cystic fibrosis (CF) and chronic obstructive pulmonary disease (COPD).

## Description

### Field of the invention

The invention relates to the treatment of inflammatory diseases, particularly inflammatory lung diseases. More particularly, it concerns oligonucleotides targeting tristetraprolin (TTP) mRNA and their use for the prevention and/or treatment of inflammatory diseases, particularly inflammatory lung diseases such as cystic fibrosis (CF) and chronic obstructive pulmonary disease (COPD).

### Background of the invention

Cystic fibrosis (CF) and COPD (chronic obstructive pulmonary disease) are both chronic lung diseases of distinct etiology, but both characterized by a decline in lung function resulting from chronic inflammation. Cystic fibrosis, a hereditary disease, is characterized by the absence or dysfunction of the CFTR channel due to the presence of mutations in the CFTR gene. The CFTR protein is responsible for chloride ion efflux at the apical membrane of epithelial cells, essential for mucus hydration and mucociliary clearance. Due to multiple cycles of infection (notably by pseudomonas aeruginosa) and inflammation, repeated lung damage is the main cause of morbidity and mortality in cystic fibrosis patients. COPD affects 3.5 million people in France (the third leading cause of death worldwide), and results from chronic exposure to cigarette smoke, air pollution and aerosols. Despite the therapies that have emerged in recent years, notably for cystic fibrosis, the chronic inflammation found in these diseases remains a major problem.

Preventing chronic inflammation by designing new therapies that diminish the pro-inflammatory response is a major area of research in cystic fibrosis (irrespective of genotype) and COPD. The altered expression of systemic inflammation players is well documented, with an increase in cytokines TNFα, IL-8 and IL-6. These pro-inflammatory cytokines are subject to various regulations during infection/inflammation, including posttranscriptional regulation by RNA-Binding protein (RNA-BP), which binds to the 3'UTR end of target mRNAs, leading to their degradation or translational inhibition.

One of these RNA-BPs, tristetraprolin (TTP or ZFP36), acts as a cytoplasmic regulator and binds, when unphosphorylated, to the 3'untranslated region (3'UTR end) of mRNAs for pro-inflammatory mediators and neutrophil chemotactics factors, notably IL-1, IL-6, IL-8 and TNFα, to control their expression. The importance of TTP has been demonstrated in mice with TTP gene invalidation (KO TTP-/-) who developed a systemic inflammatory syndrome (Carballo E. et. al., "Feedback inhibition of macrophage tumor necrosis factor-alpha production by tristetraprolin", Science, August 14, 1998; 281(5379):1001-5). Overexpression of the TTP gene in mice induces protection against the development of inflammatory diseases (Patial S. et. al., "Tristetraprolin as a therapeutic target in inflammatory disease", Trends Pharmacol Sci. 2016 b;37(10):811-21). Deletion of a portion of the 3'UTR end of TTP induces an increase in the level of TTP mRNA in mice, protecting them against Lipopolysaccharide-induced acute lung injury (Choudhary et. al., "Tristetraprolin overexpression in non-hematopoietic cells protects against acute lung injury in mice", Front Immunol, September 2, 2020; 11:2164), highlighting the possibility of targeting the 3'UTR end of TTP to induce its overexpression.

This protein therefore appears to be a promising target for controlling the inflammatory response in inflammatory diseases, particularly inflammatory lung diseases such as cystic fibrosis and COPD. Since deletion of a portion of the 3'UTR is currently unthinkable in humans (compared with the mouse model), it now appears necessary to find an alternative strategy for modulating TTP mRNA expression without affecting genome integrity.

### Summary of the invention

The aim of the present invention is to provide such a treatment.

It has been discovered by the present inventors that such objective is achieved by the implementation of particular oligonucleotides (ASO), these oligonucleotides efficiently hybridizing to the 3'UTR complementary sequence of tristetraprolin (TTP) mRNA. These oligonucleotides may commonly be referred to as target site blockers (TSBs) and will increase the level of TTP mRNA.

Thus, according to a first aspect, the present invention relates to an oligonucleotide whose nucleotide sequence comprises, or consists of, a sequence having at least 90% identity, preferably 91%, 92%, 93%, 94%, 95%, 96%, 97%, 98%, 99% or 100% identity with a sequence selected from SEQ ID NO: 1 and SEQ ID NO: 2.

When integrated into human cell cultures, the ASOs of the present invention target the 3'untranslated region (3'UTR end) of TTP mRNA and block their degradation, showing an advantageous increase in TTP mRNA and TTP protein levels. This effect is also accompanied by an advantageous decrease in pro-inflammatory mRNAs (IL8, IL6, TNFα, etc.) in cell cultures from patients suffering from cystic fibrosis (CF) and chronic obstructive pulmonary disease (COPD). The ASOs of the present invention are advantageously effective in the treatment or prevention of inflammatory diseases, particularly inflammatory lung diseases such as cystic fibrosis and COPD.

The percentage of identity between two nucleotide sequences, in the sense of the present invention, is determined by comparing the two optimally aligned sequences through a comparison window. The part of the nucleotide sequence in the comparison window may thus comprise additions or deletions (e.g. "gaps") with respect to the reference sequence (which would not comprise these additions or deletions) so as to obtain an optimal alignment between the two sequences.

The percentage of sequence identity is calculated by determining the number of positions at which a nucleotide base is identical in the two sequences being compared, then dividing the number of positions at which there is identity between nucleotide bases by the total number of positions in the comparison window, then multiplying the result by one hundred to obtain the percentage of nucleotide identity between the two sequences.

In a preferred embodiment, the present invention relates to an oligonucleotide whose nucleotide sequence comprises, or consists of, a sequence selected from SEQ ID NO: 1 and SEQ ID NO: 2.

In some embodiments, the oligonucleotide comprises at least one modification selected from phosphorothioate internucleoside linkage, methylphosphonate, peptide nucleic acids, 2'-O-methyl, 2'-O-methoxyethyl, fluoro- or carbon, methylene and locked nucleic acid (LNA).

In a preferred embodiment, the oligonucleotide comprises phosphorothioate internucleoside linkage and nucleotides that are locked nucleic acid (LNA).

According to a preferred embodiment, the present invention relates to an oligonucleotide whose nucleotide sequence is selected from SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

According to a preferred embodiment, the present invention relates to an oligonucleotide whose nucleotide sequence is selected from SEQ ID NO: 3 and SEQ ID NO: 7. These oligonucleotides of sequences SEQ ID NO: 3 and SEQ ID NO: 7 comprise specific modifications: a phosphorothioate internucleoside linkage between all nucleotides and some of their nucleotides are locked nucleic acids (LNA).

When integrated into human cell cultures, the ASOs of sequences SEQ ID NO: 3 and SEQ ID NO: 7 with their specific modifications are even more efficient in targeting the 3'untranslated region (3'UTR end) of TTP mRNA and blocking their degradation, showing the best advantageous increase in TTP mRNA and TTP protein levels. This effect is also accompanied by an advantageous decrease in pro-inflammatory mRNAs (IL8, IL6, TNFα, etc.) in cell cultures from patients suffering from cystic fibrosis (CF) and chronic obstructive pulmonary disease (COPD).

In particular embodiments, the invention further satisfies the following features, implemented separately or in each of their technically operative combinations.

According to a second aspect, the present invention relates to an ASO according to the invention, for its use as a medicament.

According to a third aspect, the present invention relates to an ASO according to the invention, for its use in the prevention and/or the treatment of an inflammatory diseases, optionally an inflammatory lung disease.

According to one embodiment, the inflammatory lung disease is selected from cystic fibrosis and chronic obstructive pulmonary disease (COPD).

According to a fourth aspect, the present invention relates to a composition comprising as an active principle, at least one ASO according to the invention.

According to one embodiment, the composition comprises as active principle a at least one ASO according to the invention, in a pharmaceutically acceptable vehicle. The vehicle for the composition according to the invention may be solid, semi-solid or liquid. It may be a diluent, an adjuvant or any other vehicle conventional in itself for the constitution of pharmaceutical compositions.

The composition according to the invention may contain one or more excipients/additives conventional in themselves for the constitution of pharmaceutical compositions, for example selected from preservatives, sweetening agents, flavoring agents, bulking agents, disintegrants, wetting agents, emulsifiers, surfactants, dispersants, lubricants, stabilizers, buffers, antibacterial agents, antifungal agents, etc., or any mixtures thereof, or any of their mixtures; and/or any compound enabling rapid, prolonged or delayed, and/or targeted, release of the active ingredient after administration to the subject.

The composition according to the invention may further contain one or more active principles other than the ASO, which active principles may or may not act synergistically with said ASO.

The composition according to the invention, in its desired form, can be prepared by any method conventional in itself for the preparation of pharmaceutical compositions.

The composition according to the invention may be in any galenic form, in particular in a form suitable for parenteral, intranasal, rectal, pulmonary or topical administration. Preferably, it is in a form suitable for oral, inhalation or intratracheal administration. Examples of such non-limiting galenical forms of the invention include granules, powder, tablets, capsules, pills, syrups, solutions or oral suspensions, etc.

The invention also relates to the therapeutic use of a composition according to the invention, as defined above, for the prevention and/or treatment of an inflammatory disease, optionally an inflammatory lung disease, in particular cystic fibrosis or COPD. This use may correspond to one or more of the characteristics described above with reference to the therapeutic use of the ASO of the present invention.

The invention is also expressed in terms of a method of treating an inflammatory disease, particularly an inflammatory lung disease, comprising administering to a subject in need thereof a therapeutically effective amount of the ASO subject of the present invention or of the composition subject of the present invention. This method may meet one or more of the characteristics described above with reference to the therapeutic use of ASO as a medicament.

The invention is also expressed in terms of a use of the ASO object of the present invention or of the composition object of the present invention for the manufacture of a medicament for the treatment of an inflammatory diseases, particularly an inflammatory lung disease. This use may contain one or more of the features presented above, implemented separately or in each of their technically operative combinations.

Administration of the ASO used according to the invention to the subject to be treated can be performed by any conventional route per se, including parenterally, for example subcutaneously, subdurally, intravenously, intramuscularly, intrathecally, intraperitoneally, intracerebrally, intraarterially or intralesionally; intranasally; rectally; pulmonally, for example by aerosol or inhalation; or even topically. Oral, inhalation or intratracheal administration is preferred.

### Description of the figures

The invention will be better understood by reading the following description, given by way of example by no means limiting, and made with reference to the figures which represent:

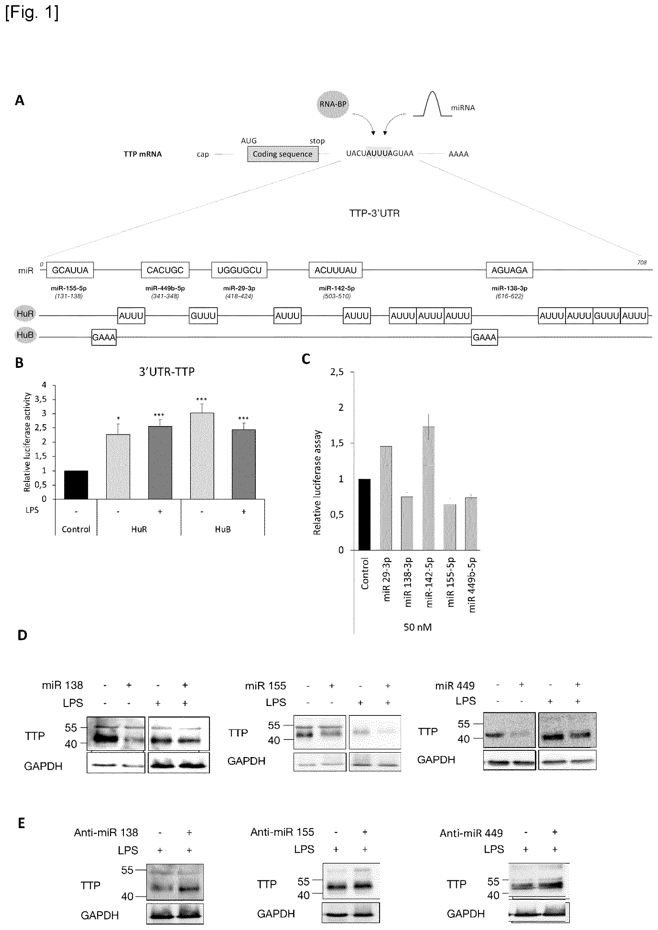

[Fig. 1] Figure 1 illustrates a drawing showing the binding sites of miRNAs and RNA-binding proteins (RNA-BPs) on TTP-3'UTR (**A**). It also illustrates the effect of the two RNA-BPs HuB and HuR in the absence or presence of LPS (10 µg/mL for 6h) on TTP-3'UTR evaluated by luciferase assay in BEAS-2B cells (**B**). It also illustrates the effect of miR-138 and miR-155 in the absence or presence of LPS (10 µg/mL for 6h) on TTP-3'UTR evaluated by luciferase assay in BEAS-2B cells (**C**). It also illustrates the effect of the miR-138, miR-449 and miR-155 in the absence or presence of LPS (10 µg/mL for 6h) on TTP protein expression in 16HBE cells (**D**). GAPDH protein level was used as loading control. It finally illustrates the effect of the anti-miR-138, anti-miR-449 and anti-miR-155 in the presence of LPS (10 µg/mL for 6h) on TTP protein expression in 16HBE-F508del cells (**E**). GAPDH protein level was used as loading control;

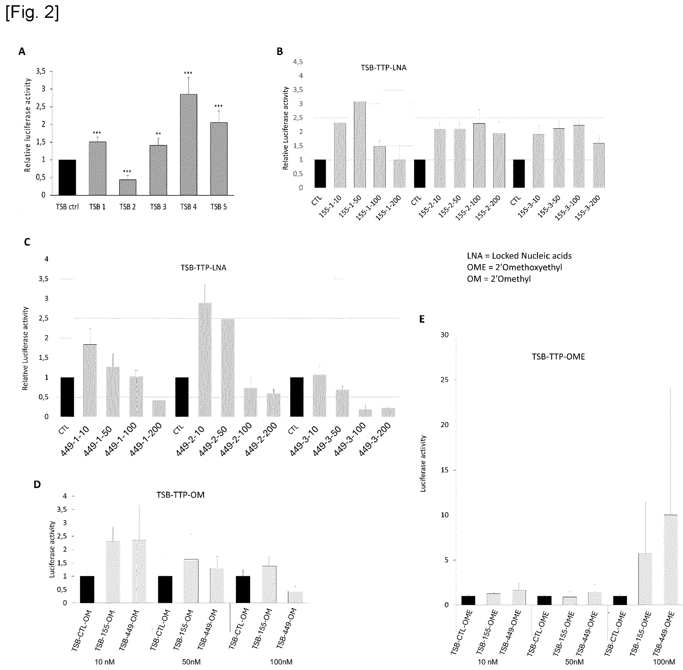

[Fig. 2] Figure 2 illustrates the effect of oligonucleotide as target site blocker (TSB) on TTP expression in bronchial cells. In particular, it illustrates the effect of different TSB (TSB 1: TSB-29, TSB 2: TSB-138, TSB 3: TSB-142, TSB 4: TSB-155 and TSB 5: TSB-449 also called TSB-449b) on TTP-3'UTR evaluated by luciferase assay in BEAS-2B cells at 50nM (**A**). It also illustrates the effect of TSB-155 with LNA modifications at different positions with phosphorothioate backbone (TSB-155-1, TSB-155-2 and TSB-155-3) at different concentrations (10, 50, 100 and 200 nM) on TTP-3'UTR evaluated by luciferase assay in BEAS-2B cells (**B**). It also illustrates the effect of TSB-449 with LNA modifications at different positions with phosphorothioate backbone (TSB-449-1, TSB-449-2 and TSB-449-3) at different concentrations (10, 50, 100 and 200 nM) on TTP-3'UTR evaluated by luciferase assay in BEAS-2B cells (**C**). It also illustrates the effect of TSB-155 and TSB-449 with 2'-O-methyl modification on each nucleotide base (TSB-155-OM, TSB-449-OM) at different concentrations (10, 50, and 100 nM) on TTP-3'UTR evaluated by luciferase assay in BEAS-2B cells (D). Finaly, it illustrates the effect of TSB-155 and TSB-449 with 2'-O-methoxyethyl modification on each nucleotide base (TSB-155-OME, TSB-449-OME) at different concentrations (10, 50, and 100 nM) on TTP-3'UTR evaluated by luciferase assay in BEAS-2B cells (**E**);

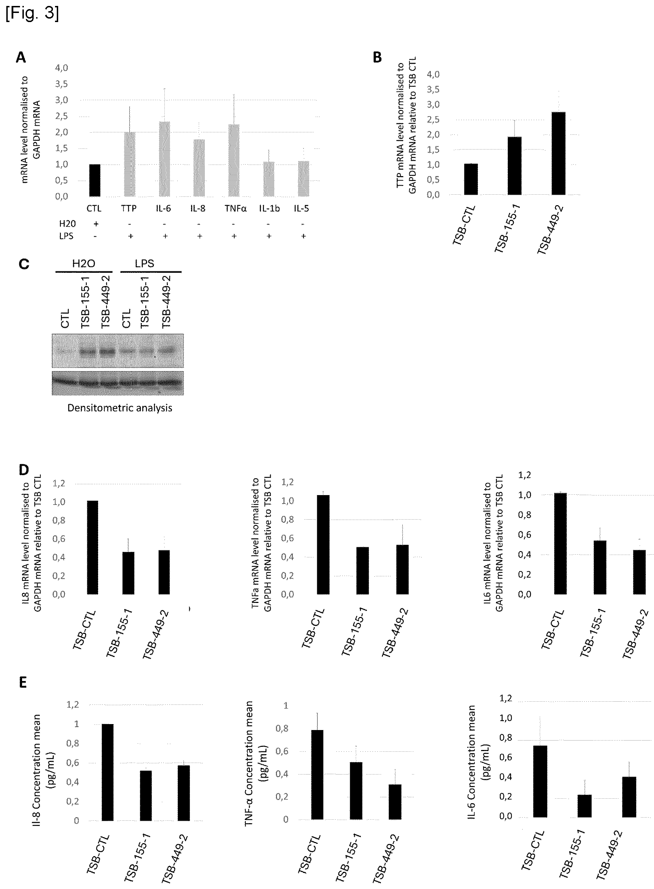

[Fig. 3] Figure 3 illustrates the TTP or cytokines (IL-6, IL-8, TNFα, IL-1b and IL-5) mRNA expression after LPS treatment (10 µg/mL) for 24h in CF cells compared to water (**A**). It also illustrates the effect of TSB-155-1 and TSB-449-2 on TTP mRNA expression in CF cells after LPS treatment (10 µg/mL) for 24h compared to the effect of a target site blocker control (TSB CTL) (**B**). It also illustrates a western blot analysis of TTP protein level after transfection of TSB-155-1 or TSB-449-2 in CF cells (**C**). Data were normalized to GAPDH protein level. It also illustrates the effect of TSB-155-1 and TSB-449-2 on cytokines mRNA expression (IL8, TNFα and IL-6) in CF cells after LPS treatment (10 µg/mL) for 24h compared to the effect of a target site blocker control (TSB CTL) (**D**). It finally illustrates the effect of TSB-155-1 and TSB-449-2 on IL8, TNFα and IL-6 cytokines secretion in CF cells after LPS treatment (10 µg/mL) for 24h by enzyme-linked immunosorbent assays (ELISAs) (**E**);

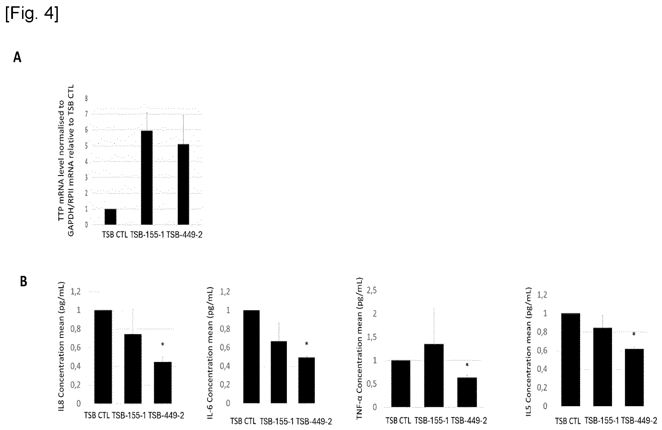

[Fig. 4] Figure 4 illustrates the effect of TSB-155-1 and TSB-449-2 on TTP mRNA expression in COPD cells after LPS treatment (10 µg/mL) and cigarette smoke extract (CSE, 10%) treatment for 24h compared to the effect of a target site blocker control (TSB CTL) (**A**). It also illustrates the effect of TSB-155-1 and TSB-449-2 on IL8, TNFα, IL-6 and IL-5 cytokines secretion in COPD cells after LPS treatment (10 µg/mL) and cigarette smoke extract (CSE, 10%) treatment for 24h compared to the effect of a target site blocker control (TSB CTL), by enzyme-linked immunosorbent assays (ELISAs) (**B**).

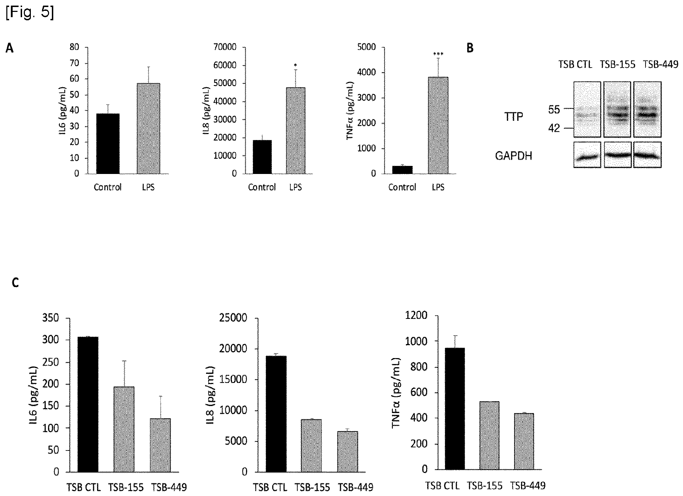

[Fig. 5] Figure 5 illustrates the effect of TSB-155-1 and TSB-449-2 on Cytokines secretion (TNFα, IL-6 and IL-8) in M1 macrophages after LPS treatment (0.1 µg/mL, for6h) compared to the non-treated M1 macrophages (named Control) (**A**). It also represents the result of a western blot analysis of TTP protein level after transfection of different TSB (TSB-155-1 and TSB-449-2) in THP-1 cells differentiated into M1 macrophages (B). Data were normalized to *GAPDH* protein level. It also illustrates the effect of TSB-155-1 and TSB-449-2 on IL8, TNFα and IL-6 cytokines secretion in M1 macrophages after LPS treatment (0.1 µg/mL) for 6h compared to the effect of a target site blocker control (TSB CTL), by enzyme-linked immunosorbent assays (ELISAs) (**C**).

### Detailed Description of the invention

The inventors have designed antisense oligonucleotides (ASOs), in particular chemically modified oligonucleotides, to hybridize to the 3'UTR sequence of the complementary TTP mRNA. These ASOs, which will also be referred to as Target Site blockers (TSBs) in this description, target 2 different sequences of the 3'UTR part of the TTP mRNA.

To validate the efficacy of these TSBs, luciferase assays were first used. These tests consisted in producing a plasmid construct (minigene) containing the Firefly luciferase cDNA upstream of the nucleotide sequence corresponding to the 3'UTR part of TTP (sequence starting after the stop codon of the TTP coding sequence, up to the poyladenylation/cleavage site, thus obtaining the 3'UTR part found on TTP mRNA). Epithelial cells of bronchial origin are then used (in 9-plate format) to introduce the LUC-3'UTR TTP plasmid and various TSBs. These TSBs are chemically modified to improve their efficacy, limit off-target side effects and avoid degradation of the target pre-mRNA in the ASO/pre-mRNA duplex by RNase H.

A significant advance in this field has been the identification of novel AON modifications (Shen et al. 2018), such as phosphate backbone or ring modifications at the base or sugar level, to improve their enzymatic stability, aqueous solubility, binding affinity and cellular uptake, and to limit immune stimulation (Dhuri et al. 2020). The ASOs subject to the invention may have a phosphorothioate (PS) backbone (Eckstein et al. 2014). The ribose sugar may contain 2'-O-methyl (OM) or 2'-O-methoxyethyl (OME) substitutions that increase oligonucleotide stability (Lamond et al. 1993). ASO modifications on sugars can also include morpholino-phosphorodiamidates, peptide nucleic acids or locked nucleic acids (LNAs), which increase targetbinding affinity and nuclease resistance (de Vrieze et al. 2021). LNAs are artificial analogues in which the ribose contains a bridge linking the 2'-O position to the 4'-C position. LNA oligonucleotides are often designed as LNA/DNA mixes (oligonucleotides with alternating LNA and DNA residues). In this class of ASOs, miravirsen (anti-miR-122), for example, is the first LNA oligonucleotide used in hepatitis patients (Janssen et al. 2013). It is an anti-miR that effectively inhibits miR-122 expression, resulting in a prolonged, dose-dependent decrease in RNA levels of hepatitis C virus (a single-stranded RNA virus that requires the presence of host hepatic miR-122 to be translated and allow virus multiplication) (Janssen et al. 2013). MRG-106 (anti-miR-155) is another LNA that targets miR-155 for the treatment of cutaneous T-cell lymphoma (Rupaimoole et al. 2017).

Using the minigene system, five distinct targets on the 3'UTR part and several modifications per target (four different) were tested (OME, OM, LNA, PS) at several concentrations, incubation times. The inventors thus identified effective TSBs and the optimal modifications. To validate the effectiveness of these TSBs in increasing TTP mRNA levels, they were introduced into cell cultures from patients and showed an increase in TTP mRNA levels, TTP proteins and a decrease in pro-inflammatory mRNAs (IL8, IL6, TNFα, etc.) in cultures from patients with cystic fibrosis (CF) and chronic obstructive pulmonary disease (COPD).

### Definitions

In the present description, the term "treatment" is taken to mean the achievement of a desired pharmacological and physiological effect. The term "treatment", as used in the present description, includes the prevention or partial prevention of one or more of the symptoms of the disease and/or the partial or total cure of the disease and/or the total or partial disappearance of one or more of its symptoms.

In the present description, "at least one" means a single compound or a mixture of several such compounds.

In the present description, "a subject in need" means a subject affected or likely to be affected by inflammatory lung disease. In particular, this subject may be a mammal, and in particular a human.

In the present description, "therapeutically effective amount" means the amount of the ASO which, when administered to a subject to treat the disease, is sufficient to ensure such treatment of the disease. The therapeutically effective amount of the ASO used according to the invention depends on a number of factors, such as the disease and its severity, the age, weight, etc., of the subject to be treated, the particular ASO used, the route and form of administration, and so on. The therapeutically effective amount of the ASO used according to the invention will be determined by a healthcare professional for each individual case. The ASO may, for example, be administered to the subject in need once or twice a day, over a long period, at regular intervals, or in a targeted manner.

In the present description, "pharmaceutically acceptable vehicle" means any vehicle which is useful for the preparation of a pharmaceutical composition and which is generally safe, non-toxic and neither biologically nor otherwise undesirable for the subject to be treated, in particular for mammals and especially humans.

As used herein, the term "mRNA" means the presently known mRNA transcript(s) of a targeted gene, and any further transcripts which may be elucidated.

As used herein, the term "miRNA" or "miR" means micro-RNA, which are small, single-stranded, non-coding RNA molecules.

By "oligonucleotide" is meant an RNA or DNA molecule that binds to another RNA or DNA (target RNA, DNA). For example, if it is an RNA oligonucleotide it binds to another RNA target by means of RNA-RNA interactions and alters the activity of the target RNA. An oligonucleotide can upregulate or downregulate expression and/or function of a particular polynucleotide.

As used herein "TTP" and "Tristetraproline" are inclusive of all family members, mutants, alleles, fragments, species, coding and noncoding sequences, sense and antisense polynucleotide strands, etc.

As used herein, the words Tristetraproline, Tristetraprolin, TTP, Zinc finger protein 36 homolog, Zinc finger protein 36, Zfp-36, ZFP36 and G0S24, GOS24, TISI 1, Growth factor-inducible nuclear protein NUP475 and GO/GI switch regulatory protein 24, NUP475, and RNFI 62A are used interchangeably in the present application.

As used herein, the term "oligonucleotide specific for" or "oligonucleotide which targets" refers to an oligonucleotide having a sequence capable of forming a stable complex with a portion of the targeted gene, or capable of forming a stable duplex with a portion of a mRNA transcript of the targeted gene. Stability of the complexes and duplexes can be determined by theoretical calculations and/or in vitro assays.

### Materials and methods

### Monolayer cell cultures

The immortalized human bronchial epithelial BEAS-2B cell line from ATCC was grown in Dulbecco's Modified Eagle Medium completed with 5% Fetal Bovine Serum (FBS), 1% Ultroser G, 1% antibiotic-antimycotic, and 1% L-glutamine.

The non-CF cells 16HBE14o- (16HBE; derived from a normal human bronchial tissue sample), the CF cells 16HBEge-p.Phe508del (16HBE-p.Phe508del : 16HBE gene edited cells for p.Phe508del) and CFBE41o-(CFBE; derived from a human Cystic Fibrosis bronchial tissue sample) cell lines were grown in Minimum Essential Medium completed with 10% FBS, 1% penicillin/streptomycin, and 1% L-glutamine.

Cystic Fibrosis (CF) or Chronic obstructive pulmonary disease (COPD) air-liquid interface (ALI) 3D cells cultures were obtained from patients suffering from these diseases. CF cells were obtained from the epithelix company and COPD cells were obtained from biopsies collected at the CHU of Montpellier.

The immortalized human monocyte THP-1 cell line was grown in RPMI 1640 medium with L-Glutamine completed with 10% FBS and 1% penicillin/streptomycin.

All cell lines were maintained at 37°C and 5% CO₂.

### Luciferase assays

BEAS-2B cells (10 000 cells/well) were co-transfected with the reporter plasmid pLenti-3'UTR-TTP (10 or 72 ng) or with pGL3c-3'UTR-IL8 (72 ng), the RNA-BP expression vectors (40 ng) and with the internal control pRL-SV40 (8 ng) that contains Renilla luciferase, as previously described (Pommier et al., 2021). Transient transfections were performed using JetPEI (Polyplus^{®}). Co-transfection with miRNAs (20 nM) and the internal control or with Target Site Blocker (TSB) (50 nM) were made by the using of Lipofectamine RNAiMAX.

A treatment with LPS from *Pseudomonas aeruginosa* (10 µg/mL), was made 6h before the cells were harvested, if not indicated otherwise.

Cells were harvested 48h after transfection, and Firefly luciferase and Renilla luciferase activities were measured using the Dual-Luciferase Reporter Assay System. Data represent the mean of at least three independent experiments with each construct tested in triplicate in each experiment.

### Transient transfections and treatments

16HBE, 16HBE-p.Phe508del and CFBE cells were plated in 6-well coated plates (200 000 cells/well). CFBE cells were transfected, after reaching 80% of confluency, with the expression vector pFRT-TTP (1 µg) using JetPEI and were treated with 10 µg/mL of LPS from *Pseudomonas aeruginosa* (L8643 - Sigma Aldrich) and with vehicle (dimethyl sulfoxide) or with 20 mM of PD98059 (ERK inhibitor) for 6h before being harvested. Transfection with 100 nM of target site blocker (TSB) were made with Lipofectamine RNAiMAX. 16HBE and 16HBE-p.Phe508del were transfected after reaching 80% of confluency, with miR (50 µM), anti-miR (100 µM) (Life Technologies SAS) or with 50 nM of TSB (IDTDNA) using Lipofectamine RNAiMAX (Life Technologies SAS). Cells were treated with 10 µg/mL of LPS from *Pseudomonas aeruginosa* for 6h before being harvested. 16HBE cells were treated 6h with 100 nM of okadaic acid (OA) from *Prorocentrum concavum* (09381 - Sigma Aldrich).

THP-1 monocytes were plated in 24-well plate (1.10⁶ cells/well) and differentiated in M0 using 150 nM of phorbol 12-myristate 13-acetate (PMA - P1585-5MG, Sigma-Aldrich) in 1 mL of medium. After 72h the medium containing PMA is removed, the cells are washed two times with warmed 1X PBS, and 1mL of medium without PMA is added. The cells were differentiated in M1 macrophages, 24h later, by addition of IFN-gamma (20ng/ml) and 1 µg/mL of LPS from *Pseudomonas aeruginosa.* Twenty-four hours later, the cells were incubated for 24h, with 50 nM of oligonucleotides target site blockers (IDTDNA), using peptides-based nanoparticles kindly provided by Prisca Boisguerin (PhyMedExp, University of Montpellier, INSERM U1046, CNRS UMR 9214) in 1 mL of new complemented medium. Oligonucleotides were thus internalized into the cells.

### RNA extraction and mRNA quantification

Total RNA was isolated with TRIzol^{™} Reagent 24h after transfection and purified by phenol-chloroform extraction. Reverse transcription (RT) was performed using 1mg of total RNA and the M-MLV reverse transcriptase (Fisher Scientific, Illkirch, France) following the manufacturer's protocol. Quantitative real time PCR (qPCR) was performed with a 1:10 dilution of first strand DNA, primers specific for each mRNA (sequences in **Table 1**) and SYBR Green mix. RT-qPCR assays were performed on a Roche LC480 LightCycler instrument. The relative expression levels were calculated using the comparative DDCt method with *RPII* and *GAPDH* as controls. The expression of the mRNAs of interest was determined using the 2(- ΔΔCt) method.

### Assessment of protein expression by western blotting

The whole proteins were extracted using 1X RIPA lysis buffer 48h after transfection and were separated on 10% SDS polyacrylamide gels. After electrophoresis at 150 or 200 V and 27 or 55 mA for 1h30, proteins were electrically transferred to PVDF membranes (Immobilon-P, 0.45 mm, Millipore S.A.S, Molsheim, France). Membranes were blocked with 5% skimmed milk in PBS/0.1% Tween-20 (T-PBS) at room temperature for 1h and then incubated with primary antibodies at 4°C overnight. After three washes in T-PBS, membranes were incubated with horseradish peroxidase (HRP)-conjugated goat anti-rabbit IgG secondary antibodies at room temperature for 1h and washed three times with T-PBS. Proteins were revealed using the Immobilon^{™} Western Chemiluminescent HRP Substrate and the Azure Sapphire^{™} Biomolecular Imager. Primary-antibodies used: anti-GAPDH (FL-335) (sc-25778, Santa Cruz Biotechnology, dilution 1:1000^{e}) and anti-Tristetraprolin (#ABE285, Millipore, dilution 1:1000^{e})

### Measurement of cytokine secretion levels from cell culture supernatant by Enzyme-Linked Immunosorbent Assay (ELISA)

Level of TNFα in the 16HBE-F508del cell culture supernatant were determined by enzyme-linked immunosorbent assays (ELISAs) following the Diaclone ELISA kit instruction. The standard solutions were used to construct standard curves. Absorbance at 450 nm was read using the Tecan Sunrise microplate reader and TNFα concentration was calculated, according to the standard curves.

### Bioinformatic analysis

For miRNA or RNA binding motif prediction, the TargetScan (www.targetscan.org) or RBPDB database (rbpdb.ccbr.utoronto.ca/), respectively, was used, and only the most conserved binding sites were considered.

### Statistical analysis

Luciferase and RT-qPCR assays were carried out at least three times (n >3 independent experiments) and each experiment was analyzed in triplicate. P values were determined using Student t-test or the non-parametric Wilcoxon (unpaired, Mann-Whitney) test for luciferase or RT-qPCR assays, respectively (*p ≤0.05). Each western blot experiment was performed at least twice.

### Experiments

### Identification of regulatory elements that bind to TTP-3'UTR extremity

In silico analysis (TargetScan and RBPDB databases) of the 3'UTR end of TTP mRNA determined the presence of miRNA and RNA-Binding Protein (RNA-BP) recognition motifs. It was assessed the impact of RNA-BP HuR and HuB which are predicted to bind to TTP-3'UTR in absence or presence of LPS (10 µg/mL for 6h). **Figure 1A** shows an illustration of the binding sites of miRNAs (miR) and RNA-BPs on TTP-3'UTR.

It was co-transfected in bronchial cells treated, a reporter vector system constituted of luciferase gene controlled by *TTP*-3'UTR with the expression vector of HuR or HuB. Luciferase activity was then observed. Luciferase activity analysis indicated that both RNA-BPs positively regulate TTP transcription via its 3'UTR, with or without LPS (**Figure 1B**).

The effect of some miRNAs (50 nM) that are dysregulated in Cystic Fibrosis or involved in inflammasome, was then tested in BEAS-2B cells using a luciferase reporter assay under the control of the 3'UTR of TTP mRNA. The miR-138, miR-155 and miR-449b (miR-449) have a negative effect on TTP luciferase activity while miR29 and miR142 have a positive effect on TTP luciferase activity (**Figure 1C**).

Then the impact of the miR-138, miR-155 and miR-449 on TTP protein level (upper and lower panel respectively) in 16HBE cells was analyzed. With the three miRNAs, there is a decrease of TTP protein level compared to control which is maintained with LPS treatment (**Figure 1D**). To test if it is possible to increase TTP protein level, miR-138, miR-155 or miR-449 inhibitors (respectively Anti-miR-138, Anti-miR-155 or Anti-miR-449) were transfected into CF cells (16HBE-F508del) treated with LPS. The inhibition of those miRNAs increased TTP protein level (**Figure 1E**). These findings suggest that TTP expression can be regulated by the modulation of its regulatory elements that bind on its 3'UTR extremity.

### Effect of oligonucleotide as target site blocker (TSB) on TTP expression in bronchial cells

The inventors designed oligonucleotides acting as target site blocker (TSB) to prevent the binding of repressive trans-regulators on TTP-3'UTR, to instore a strategy to increase TTP expression. These TSB are called TSB 1 (or TSB 29), TSB 2 (or TSB 138), TSB 3 (or TSB 142), TSB 4 (or TSB 155) and TSB 5 (or TSB 449b or TSB 449) and respectively target the sites of miR-29, miR-138, miR-142, miR-155 and miR-449. The sequences of the oligonucleotides (TSB) are given in **Table 1.** Co-transfection of TSB 1, TSB 3, TSB 4 and TSB 5 (50 nM) with pLenti-3'UTR-TTP reporter vector in bronchial cells (BEAS-2B cells) led to a 1.5- to 3-fold increase of luciferase activity, whereas the TSB 2 decreased it (**Figure 2A**). Target site blocker of sequence SEQ ID NO: 14 was used as control (TSB-ctrl).

Then, the effect of TSB-155 with LNA modifications at different positions with phosphorothioate backbone (TSB-155-1, TSB-155-2 and TSB-155-3) at different concentrations (10, 50, 100 and 200 nM) on TTP-3'UTR was evaluated by luciferase assay in BEAS-2B cells, and the results are illustrated in **Figure 2B****.** The sequences of the oligonucleotides with their different modifications are given in **Table 1.** Target site blocker of sequence SEQ ID NO: 14 was used as control (CTL).

The results showed that TSB-155-1 (10 and 50nM), TSB-155-2 (10, 50, 100 and 200 nM) and TSB-155-3 (10, 50 and 100nM) stabilized the 3'UTR of TTP mRNA by 2.1 to 3.1 fold.

The effect of TSB-449 with LNA modifications at different positions with phosphorothioate backbone (TSB-449-1, TSB-449-2 and TSB-449-3) at different concentrations (10, 50, 100 and 200 nM) on TTP-3'UTR was also evaluated by luciferase assay in BEAS-2B cells, and the results are illustrated in **Figure 2C****.** The sequences of the oligonucleotides with their different modifications are given in **Table 1.** Target site blocker of sequence SEQ ID NO: 14 was used as control (CTL).

The results showed that TSB-449-1 (10 nM), TSB-449-2 (10 and 50 nM) stabilized the 3'UTR of TTP mRNA by 1.8 to 3.2 fold.

Then, the effect of TSB-155 and TSB-449 with 2'-O-methyl modification on each nucleotide base (TSB-155-OM, TSB-449-OM) at different concentrations (10, 50, and 100 nM) on TTP-3'UTR was evaluated by luciferase assay in BEAS-2B cells, and the results are illustrated in **Figure 2D****.** A target site blocker of sequence SEQ ID NO: 12 is used as control (TSB-CTL-OM). The sequences of the oligonucleotides with their 2'-O-methyl modification are given in **Table 1.**

The results showed that TSB-155-OM and TSB-449-OM stabilized the 3'UTR of TTP mRNA by 2.3 fold.

The effect of TSB-155 and TSB-449 with 2'-O-methoxyethyl modification on each nucleotide base (TSB-155-OME, TSB-449-OME) at different concentrations (10, 50, and 100 nM) on TTP-3'UTR was evaluated by luciferase assay in BEAS-2B cells, and the results are illustrated in **Figure 2E****.** A target site blocker of sequence SEQ ID NO: 13 is used as control (TSB-CTL-OME). The sequences of the oligonucleotides with their 2'-O-methoxyethyl modification are given in **Table 1.**

The results showed that TSB-155-OME and TSB-449-OME stabilized the 3'UTR of TTP mRNA at high concentration (100nM).

### Impact of oligonucleotide as target site blocker (TSB) on TTP and cytokine expression in CF cells

The TTP or cytokines (IL-6, IL-8, TNFα, IL-1b and IL-5) mRNA expression was observed after LPS treatment (10 µg/mL) for 6h in CF cells compared to water (H₂O) following the mRNA quantification method given in the materials and methods part above. The results are shown in **Figure 3A****.**

The results validated that LPS stimulated cytokines secretion.

Then, the effect of TSB-155-1 and TSB-449-2 on TTP mRNA expression in CF cells was observed after LPS treatment (10 µg/mL) for 6h compared to the effect of the target site blocker control of sequence SEQ ID NO:14 (TSB CTL) following the mRNA quantification method given in the materials and methods part above. The results are shown in **Figure 3B****.**

The results showed that TSB-155-1 and TSB-449-2 induced an increase in the TTP mRNA level in CF epithelium cultured in ALI.

This was followed by a western blot analysis of TTP protein level after transfection of TSB-155-1 or TSB-449-2 in CF cells following the method given in the assessment of protein expression by western blotting paragraph of the materials and methods part above. Data were normalized to GAPDH protein level. The results are shown in **Figure 3C****.** The target site blocker of sequence SEQ ID NO:14 was used as control (CTL).

The results showed that TSB-155-1 and TSB-449-2 induced an increase in the TTP protein level in CF epithelium cultured in ALI.

After that, the effect of TSB-155-1 and TSB-449-2 on cytokines mRNA expression (IL8, TNFα and IL-6) in CF cells after LPS treatment (10 µg/mL) for 6h was tested compared to the effect of the target site blocker control of sequence SEQ ID NO:14 (TSB CTL). The results are shown in **Figure 3D****.**

The results showed that TSB-155-1 and TSB-449-2 induced a decrease in the cytokines mRNA level (IL6, IL8 and TNFa) in CF epithelium cultured in ALI.

It was then tested the effect of TSB-155-1 and TSB-449-2 on IL8, TNFα and IL-6 cytokines secretion in CF cells after LPS treatment (10 µg/mL) for 6h by enzyme-linked immunosorbent assays (ELISAs) following the method of measurement of cytokine secretion levels in the materials and methods part above. The results are shown in **Figure 3E****.** The target site blocker of sequence SEQ ID NO:14 was used as control (TSB-CTL).

The results showed that TSB-155-1 and TSB-449-2 induced a decrease in the cytokines secretion (IL6, IL8 and TNFa) in CF epithelium cultured in ALI.

### Impact of oligonucleotide as target site blocker (TSB) on TTP expression and cytokine secretion in COPD cells

The effect of TSB-155-1 and TSB-449-2 on TTP mRNA expression in COPD cells was observed after LPS treatment (10 µg/mL) and cigarette smoke extract (CSE, 10%) treatment for 6h compared to the effect of the target site blocker control of sequence SEQ ID NO:14 (TSB CTL) following the mRNA quantification method given in the materials and methods part above. The results are shown in **Figure 4A****.**

The results showed that TSB-155-1 and TSB-449-2 induced an increase in the TTP mRNA level in COPD epithelium cultured in ALI.

Then, the effect of TSB-155-1 and TSB-449-2 on IL8, TNFα, IL-6 and IL-5 cytokines secretion in COPD cells was observed after LPS treatment (10 µg/mL) and cigarette smoke extract (CSE, 10%) treatment for 6h compared to the effect of the target site blocker control of sequence SEQ ID NO:14 (TSB CTL) by enzyme-linked immunosorbent assays (ELISAs) following the method of measurement of cytokine secretion levels in the materials and methods part above. The results are shown in **Figure 4B****.**

The results showed that TSB-155-1 and TSB-449-2 induced a decrease in the cytokines secretion (IL5, IL6, IL8 and TNFa) in COPD epithelium cultured in ALI.

### Impact of oligonucleotide as target site blocker (TSB) on TTP expression and cytokine secretion in macrophages M1.

The response of IL-6, IL-8 and TNFα cytokines secretion to LPS was observed in M1 macrophages after LPS treatment (0.1 µg/mL, for 6h) compared to the non-treated M1 macrophages (named Control) The results are shown in **Figure 5A****.**

The results showed that LPS treatment induced an increase in secretion of IL-6, IL-8 and TNFα cytokines in macrophages M1-derived THP-1 cells.

This was followed by a western blot analysis of TTP protein level after transfection of TSB-155-1 (TSB 155) or TSB-449-2 (TSB 449) in macrophages M1-derived THP-1 cells following the method given in the assessment of TTP protein expression by western blotting paragraph of the materials and methods part above. Data were normalized to GAPDH protein level. The results are shown in **Figure 5B****.** Target site blocker of sequence SEQ ID NO:14 was used as control (TSB CTL).

The results showed that TSB-155-1 and TSB-449-2 induced an increase in the TTP protein level in macrophages M1-derived THP-1 cells.

Then, the effect of TSB-155-1 (TSB 155) and TSB-449-2 (TSB 449) on IL8, TNFα and IL-6 cytokines secretion in macrophages M1-derived THP-1 cells was observed after LPS treatment (0.1 µg/mL) for 6h compared to the effect of the target site blocker control of sequence SEQ ID NO:14 (TSB CTL) by enzyme-linked immunosorbent assays (ELISAs) following the method of measurement of cytokine secretion levels in the materials and methods part above. The results are shown in **Figure 5C**.

The results showed that TSB-155-1 and TSB-449-2 induced a decrease in the cytokines secretion (IL6, IL8 and TNFa) in macrophages M1-derived THP-1 cells.

### [Table 1]

**Table 1: Sequences cited in the present specification (* = Phosphorothioate, m = 2'Omethyl (OM), 2MOE = 2'Omethoxyethyl (OME), + = Locked Nucleic acids (LNA)).**

| **SED ID NO**. | **Sequence** | **Description** |
|---|---|---|
| **1** | GAGTGGGTTAATGCAT | ASO of the present invention without modifications, targeting the 3'UTR end of TTP mRNA (TSB 4) |
| **2** | GAGGGGCAGTGAAGGC | ASO of the present invention without modifications, targeting the 3'UTR end of TTP mRNA (TSB 5) |
| **3** | +G*A*+G*T*+G*G*+G*T*+T*A*+A*T*+G*C*+A*T | TSB155-1 |
| **4** | G*A*+G*T*G*+G*G*T*+T*A*A*+T*G*C*+A*T | TSB155-2 |
| **5** | +G*A*G*+T*G*G*+G*T*T*+A*A*T*+G*C*A*+T | TSB155-3 |
| **6** | +G*A*+G*G*+G*G*+C*A*+G*T*+G*A*+A*G*+G*C | TSB449-1 |
| **7** | G*A*+G*G*G*+G*C*A*+G*T*G*+A*A*+G*G*+C | TSB449-2 |
| **8** | mG*mA*mG*mU*mG*mG*mG*mU*mU*mA*mA* mU*mG*mC*mA*mU | TSB155-OM |
| **9** | mG*mA*mG*mG*mG*mG*mC*mA*mG*mU*mG* mA*mA*mG*mG*mC | TSB449-OM |
| **10** | 2MOEG*2MOEA*2MOEG*2MOET*2MOEG*2MOEG *2MOEG*2MOET*2MOET*2MOEA*2MOEA*2MOET * 2MOEG*2MOEC*2MOEA*2MOET | TSB155-OME |
| **11** | 2MOEG*2MOEA*2MOEG*2MOEG*2MOEG*2MOEG *2MOEC*2MOEA*2MOEG*2MOET*2MOEG*2MOEA *2MOEA*2MOEG*2MOEG*2MOEC | TSB449-OME |
| **12** | mU*mA*mA*mC*mA*mC*mG*mU*mC*mU*mA*m U*mA*mC*mG*mC*mC*mC*mA | TSB control (TSB-CTL-OM) in evaluation of the effect of TSB-155-OM and TSB-449-OM with 2'-O-methyl modification on TTP-3'UTR (TSB ctrl in figure 2D) |
| **13** | 2MOET*2MOEA*2MOEA*2MOEC*2MOEA*2MOEC* 2MOEG*2MOET*2MOEC*2MOET*2MOEA*2MOET* 2MOEA*2MOEC*2MOEG*2MOEC*2MOEC*2MOEC* 2MOEA | TSB control (TSB-CTL-OME) in evaluation of the effect of TSB-155-OME and TSB-449-OME with 2'-O-methoxyethyl modification on TTP-3'UTR (TSB ctrl in figure 2E) |
| **14** | T*A*+A*C*A*+C*G*T*+C*T*A*+T*A*C*+G*C*C*+C *A | TSB control in evaluation of the effect of different TSB on TTP-3'UTR (TSB ctrl in figure 2A, CTL in figures 2B, 2C, and TSB CTL in figure 3, 4 and 5) |
| **15** | A*T*T*T*G*A*G*C*A*C*C*A*G*G*A*T | TSB 1 |
| **16** | T*A*G*T*C*T*A*C*T*T*A*T*A*A*T*A*A*T*T*A*T *A*T*C*G*T*C | TSB 2 |
| **17** | A*A*A*T*A*A*A*G*T*C*G*T*C*A*T*A | TSB 3 |

## Claims

1. An oligonucleotide whose nucleotide sequence comprises, or consists of, a sequence having at least 90% identity, preferably 91%, 92%, 93%, 94%, 95%, 96%, 97%, 98%, 99% or 100% identity with a sequence selected from SEQ ID NO: 1 and SEQ ID NO: 2.

2. An oligonucleotide according to claim 1, wherein its nucleotide sequence comprises, or consists of, a sequence selected from SEQ ID NO: 1 and SEQ ID NO: 2.

3. An oligonucleotide according to claim 1 or 2, comprising at least one modification selected from phosphorothioate internucleoside linkage, methylphosphonate, peptide nucleic acids, 2'-O-methyl, 2'-O-methoxyethyl, fluoro- or carbon, methylene and locked nucleic acid (LNA).

4. An oligonucleotide according to claim 3, comprising phosphorothioate internucleoside linkage and nucleotides that are locked nucleic acid (LNA).

5. An oligonucleotide according to anyone of claim 1 to 4, wherein its nucleotide sequence is selected from SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and SEQ ID NO: 11.

6. An oligonucleotide according to claim 5, wherein its nucleotide sequence is selected from SEQ ID NO: 3 and SEQ ID NO: 7.

7. An oligonucleotide according to anyone of claim 1 to 6, for its use as a medicament.

8. An oligonucleotide according to anyone of claim 1 to 6, for its use in the prevention and/or the treatment of an inflammatory disease.

9. An oligonucleotide according to anyone of claim 1 to 7, for its use in the prevention and/or the treatment of an inflammatory lung disease

10. An oligonucleotide according to claim 9, wherein the inflammatory lung disease is selected from cystic fibrosis and chronic obstructive pulmonary disease (COPD).

11. Composition comprising as an active principle, at least one ASO according to anyone of claim 1 to 6.

12. Composition according to claim 11, wherein said composition is in any galenic form, preferably in a form suitable for parenteral, oral, intranasal, rectal, pulmonary or topical administration.

13. Composition according to claim 11 or 12, for its use in the prevention and/or the treatment of an inflammatory disease, optionally an inflammatory lung disease such as cystic fibrosis or chronic obstructive pulmonary disease.
